# EUROPÄISCHE PATENTSCHRIFT

(11) **EP 1 680 680 B1**
(45) Veröffentlichungstag und Bekanntmachung des Hinweises auf die Patenterteilung: **10.10.2007**
(21) Anmeldenummer: 04791020.3
(22) Anmeldetag: 29.10.2004
(51) Int. Cl.: G01N 33/68, G01N 33/92, G01N 33/94

(54) **DIAGNOSESTELLUNG VON AKUTEN MYOKARDIALEN, ISCHÄMISCHEN ERKRANKUNGEN DURCH KOMBINATION VON MARKERN**
DIAGNOSIS OF ACUTE MYOCARDIAL, ISCHAEMIC DISEASES BY MEANS OF A COMBINATION OF MARKERS
ETABLISSEMENT DE DIAGNOSTIC DE MALADIES AIGUES ISCHEMIQUES ET DU MYOCARDE PAR COMBINAISON DE MARQUEURS

(30) Priorität: 03.11.2003 DE 10351238
(43) Veröffentlichungstag der Anmeldung: 19.07.2006
(73) Patentinhaber: F. HOFFMANN-LA ROCHE AG, 4070 Basel (CH); Roche Diagnostics GmbH, 68305 Mannheim (DE)
(72) Erfinder: AMANN-ZALAN, Ildiko, 69469 Weinheim (DE); SPINKE, Jürgen, 64653 Lorsch (DE)
(74) Vertreter: Weiss, Wolfgang
(86) Internationale Anmeldenummer: PCT/EP2004/012259
(87) Internationale Veröffentlichungsnummer: WO 2005/043169

(56) Entgegenhaltungen:
- EP-A2- 1 363 128
- WO-A1-02/23191
- US-A1- 2003 109 420
- US-B1- 6 534 323
- SABATINE MARC S ET AL: "Multimarker approach to risk stratification in non-ST elevation acute coronary syndromes: Simultaneous assessment of troponin I, C-reactive protein, and B-type natriuretic peptide" CIRCULATION, AMERICAN HEART ASSOCIATION, DALLAS, TX, US, Bd. 105, Nr. 15, 16. April 2002 (2002-04-16), Seiten 1760-1763, XP002262207 ISSN: 0009-7322
- MAIR J ET AL: "THE IMPACT OF CARDIAC NATRIURETIC PEPTIDE DETERMINATION ON THE DIAGNOSIS AND MANAGEMENT OF HEART FAILURE" CLINICAL CHEMISTRY AND LABORATORY MEDICINE, WALTER DE GRUYTER UND CO, DE, Bd. 39, Nr. 7, Juli 2001 (2001-07), Seiten 571-588, XP008024871 ISSN: 1434-6621

## Beschreibung

Die vorliegende Erfindung betrifft ein Verfahren zur Diagnose von akuten myokardialen, ischämischen Erkrankungen, beispielsweise akutem Myokardinfarkt, insbesondere ohne ST-Streckenhebung im EKG (NSTEMI), wobei eine Bestimmung von mindestens zwei Markern an einem zu untersuchenden Patienten durchgeführt wird. Weiterhin wird ein Kit zur Durchführung des Diagnoseverfahrens, insbesondere ein Teststreifen zur Durchführung von Schnelltests bereitgestellt.

Die Diagnose des akuten Myokardinfarkts wird derzeit aufgrund von drei Kriterien erstellt: Vorhandensein von EKG-Veränderungen, Brustschmerz und abnormal erhöhten kardialen Enzymen. Dennoch ist eine zuverlässige Diagnose oft schwierig, da 60 % der Patienten mit akutem Myokardinfarkt keine Veränderungen im EKG aufweisen und 33 % der Patienten keine typischen Brustschmerzen. Daher wird zur Diagnose des akuten Myokardinfarkts (insbesondere bei NSTEMI) vermehrt die Bestimmung von kardiospezifischen Markern herangezogen, wie etwa Troponin T und B-Typ-natriuretischem Peptid (BNP). Die Erhöhung der Konzentration eines dieser Marker ist mit einer Erhöhung der Wahrscheinlichkeit von ischämischen Ereignissen einschließlich Tod verbunden. Dies ist beispielsweise in den Veröffentlichungen Hamm et al., (New Engl. J. Med. 327 (1992), 146-150), Hamm et al., (New Engl. J. Med. 340 (1999), 1623-1629, Heeschen et al. (The Lancet 354 (1999), 1757-1962), Klootwijk und Hamm (The Lancet 353, Suppl. II (1999), 10-15), Wei et al. (Circulation 88 (1993), 1004-1009), De Lemos (New Engl. J. Med. 345 (2001), 1014-1021) beschrieben.

Bei De Winter et al. (Cardiovasc. Res. 42 (1999), 240-245) und De Winter et al. (Clin. Chem. 46 (2000), 1597-1603) wird bereits festgestellt, dass CRP und Troponin I bzw. Troponin T zwei unabhängige Marker für die Risikostratifizierung von Patienten mit akutem Koronarsyndrom sind.

In EP 03 010 818.7 wird ein Verfahren zur Diagnose von Myokardinfarkt oder/und zur Risikostratifizierung des akuten Koronarsyndroms beschrieben, bei dem eine Bestimmung von mindestens drei unterschiedlichen kardiospezifischen Markern erfolgt, wobei jeweils mindestens ein neurohormonaler Marker, mindestens ein kardiospezifischer ischämischer Marker, wie etwa Troponin T oder Troponin I, und mindestens ein inflammatorischer Marker, wie etwa C-reaktivem Protein (CRP), bestimmt wird.

Das US-Patent 6 461 828 offenbart die Verwendung einer Kombination von kardiospezifischen chemischen Markern, wie Troponin I und T zusammen mit kardiospezifischen neurohormonalen Markern, wie ANP, ProANP, BNP und ProBNP zur Diagnose und Risikostratifizierung von Patienten, die an kongestivem Herzversagen (CHF) leiden.

WO 02/089657 offenbart ein Verfahren zur Diagnose einer myokardialen Ischämie, wobei als Marker BNP oder ein mit BNP verwandter Marker, z.B. ProBNP, und gegebenenfalls ein weiterer diagnostischer Marker, wie etwa Creatinkinase, z.B. CK-MB bestimmt wird.

WO 2004/059293 (publiziert am 15.07.2004) betrifft diagnostische Verfahren, u.a. ein Verfahren zur differenziellen Diagnose von Herzversagen und arterieller Fibrillierung, wobei BNP oder ein verwandtes Peptid, wie ProBNP, Troponin I oder/und Troponin T, Myoglobin oder/und CK-MB, als Marker bestimmt werden.

Nachteilig an bekannten Diagnoseverfahren ist jedoch, dass eine frühzeitige Erfassung von Risikopatienten nicht zuverlässig gelingt. So findet ein Anstieg von kardiospezifischen ischämischen Parametern, wie etwa Troponin T oder/und I, erst relativ spät (im Allgemeinen ca. 4 Stunden) nach Auftreten eines akuten koronaren Ereignisses statt. Im Falle, dass der Patient zu diesem Zeitpunkt unklare klinische Symptome bzw. EKG-Befunde hat, kann somit erst zu einem relativ späten Zeitpunkt eine eindeutige Diagnose und eine Therapieeinleitung erfolgen. Andere ischämische Marker, wie etwa ischämisch modifiziertes Albumin (IMA) oder muskelspezifische Marker, wie etwa Myoglobin, sind zwar zu einem früheren Zeitpunkt erhöht, weisen jedoch eine nur vergleichsweise niedrige Kardiospezifität auf. Neurohormonale Marker, wie ProBNP, sind nach derzeitigem Kenntnisstand nicht ischämiespezifisch, obwohl sie bei Myokardinfarkt eine hohe Kardiospezifität aufweisen.

Eine der Erfindung zugrunde liegende Aufgabe bestand somit darin, ein Verfahren zur Diagnose von akuten myokardialen, ischämischen Erkrankungen, beispielsweise von akutem Myokardinfarkt, zu entwickeln, das eine frühere und bessere Erfassung von akuten koronaren Ereignissen ermöglicht.

Diese Aufgabe wird dadurch gelöst, dass eine Bestimmung von mindestens zwei Markern an einem zu untersuchenden Patienten durchgeführt wird, wobei mindestens ein kardiospezifischer neurohormonaler Marker und ischämisch modifiziertes Albumin als ein nicht-kardiospezifischer ischämischer Marker und gegebenenfalls ein nicht-kardiospezifischer muskelspezifischer Marker bestimmt wird. Gegebenenfalls kann darüber hinaus eine zusätzliche Bestimmung von weiteren Markern, insbesondere von mindestens einem nicht-kardiospezifischen Marker für die Plättchenaktivierung oder/und einem kardiospezifischen ischämisch-nekrotischen Marker erfolgen.

Überraschenderweise wurde festgestellt, dass die gemeinsame Bestimmung eines kardiospezifischen neurohormonalen Markers mit mindestens einem der genannten nicht-kardiospezifischen Marker zu einer verbesserten Früherkennung von akuten koronaren Ereignissen führt und damit eine Diagnose bzw. Prognose noch vor Anstieg von kardiospezifischen ischämischen Parametern erlaubt. Damit können unnötige Hospitalisierungen oder länger dauernde Aufenthalte in der Notaufnahme bzw. auf der Intensivstation vermieden werden.

Ein sich daraus ergebender Vorteil ist, dass bei Vorliegen akuter kardialer Ereignisse, beispielsweise akutem Myokardinfarkt, insbesondere bei NSTEMI, eine höhere Anzahl von Patienten zu einem früheren Zeitpunkt identifiziert und adäquat behandelt werden kann als beim gegenwärtigen diagnostischen Vorgehen. Durch die erfindungsgemäße Kombination von mindestens zwei unterschiedlichen Markern bei der Diagnosestellung kann somit die Häufigkeit von Todesfällen und anderen kardialen Komplikationen reduziert werden.

Unter "kardiospezifisch" wird im Sinne der vorliegenden Erfindung ein Marker verstanden, dessen Erhöhung in einem eindeutigen Zusammenhang mit einer kardialen Erkrankung steht und der hauptsächlich aus dem Herzen sezerniert wird. Im Gegensatz dazu steht ein "nicht-kardiospezifischer" Marker nicht in eindeutigem Zusammenhang mit einer kardialen Erkrankung und kann auch bei nicht-kardialen Erkrankungen erhöht sein.

Das erfindungsgemäße Verfahren umfasst die Bestimmung von mindestens zwei Markern, wobei mindestens ein kardiospezifischer neurohormonaler Marker und ischämisch modifiziertes Albumin (IMA) als ein nicht-kardiospezifischer ischämischer Marker und gegebenenfalls mindestens ein nicht-kardiospezifischer muskelspezifischer Marker bestimmt wird.

Der neurohormonale Marker kann beispielsweise ausgewählt werden aus atrialem (A-Typ) natriuretischem Peptid (ANP), B-Typ natriuretischem Peptid (BNP) oder N-terminalen Fragmenten der jeweiligen Propeptide NT-ProANP und NT-ProBNP. Besonders bevorzugt wird BNP oder NT-ProBNP als neurohormonaler Marker bestimmt.

Der nicht-kardiospezifische ischämische Marker ist ischämisch modifiziertes Albumin (IMA).

Als bevorzugte nicht-kardiospezifische muskelspezifische Marker können beispielsweise Myoglobin oder/und Creatinkinase-MB (CK-MB) bestimmt werden. Besonders bevorzugt wird Myoglobin oder CK-MB als nicht-kardiospezifischer muskelspezifischer Marker bestimmt.

Weiterhin kann beim erfindungsgemäßen Verfahren noch zusätzlich mindestens ein nicht-kardiospezifischer Marker für die Plättchenaktivierung bestimmt werden. Besonders bevorzugt wird CD40 als Marker für die Plättchenaktivierung bestimmt.

Darüber hinaus kann beim erfindungsgemäßen Verfahren noch zusätzlich mindestens ein kardiospezifischer ischämisch-nekrotischer Marker bestimmt werden. Als kardiospezifische ischämisch-nekrotische Marker können beispielsweise Troponin T oder Troponin 1 bestimmt werden. Besonders bevorzugt wird Troponin T als kardiospezifischer ischämisch-nekrotischer Marker bestimmt.

Die erfindungsgemäße Kombinationsbestimmung wird vorzugsweise so durchgeführt, dass an einer oder mehreren Proben aus einem zu untersuchenden Patienten parallele Bestimmungen der Marker durchgeführt werden. Eine positive Diagnose auf das Vorhandensein einer akuten Erkrankung ergibt sich aus einer positiven Diagnose für mindestens einen der getesteten Marker und vorzugsweise aus einer positiven Diagnose für mindestens zwei Marker. Durch die Kombination der angegebenen Marker kann sowohl die Spezifität als auch die Sensitivität des Tests erhöht werden.

Vorzugsweise werden eine oder mehrere aus dem Patienten stammende Proben, beispielsweise Blut- oder Serumproben, in einem oder mehreren Tests gleichzeitig oder unmittelbar hintereinander untersucht. Besonders bevorzugt werden die Bestimmungen an einer einzigen Patientenprobe durchgeführt.

Die kombinierte Bestimmung der Marker kann grundsätzlich nach beliebigen bekannten Methoden mit marktgängigen kommerziellen Tests durchgeführt werden. Zur Bestimmung können beispielsweise Analyseautomaten eingesetzt werden. Alternativ können auch Schnelltests, beispielsweise für die Anwendung in der Notaufnahme, in der Station oder Intensivstation, in der Ambulanz oder Arztpraxis oder als Patientenselbsttest eingesetzt werden.

Die Bestimmung der Marker erfolgt üblicherweise durch einen Immunoassay unter Verwendung von gegen die Marker gerichteten Antikörpern. Bevorzugt ist die Bestimmung auf einem oder mehreren Teststreifen, auf denen die zur Bestimmung der einzelnen Marker verwendeten Reagenzien in trockener - und nach Kontakt mit der Probe auflösbarer - Form in einer oder mehreren Zonen angeordnet sind, und wobei der Nachweis der einzelnen Parameter in einer Nachweiszone, vorzugsweise jeweils in separaten Bereichen der Nachweiszone erfolgt. Besonders bevorzugt werden mehrere Marker auf einem einzigen Teststreifen bestimmt.

Darüber hinaus kann die Bestimmung der Marker selbstverständlich auch in Flüssigtests erfolgen.

Der Nachweis von BNP oder NT-ProBNP als neurohormonaler Marker ist beispielsweise bei Richards et al. (Circulation 97 (1998), 1921-1929), Struthers (Eur. Heart J. 20 (1999), 1374-1375), Hunt et al. Clin. Endocrinol. 47 (1997), 287-296), Talwar et al. (Eur. Heart J. 20 (1999), 1736-1744), Darbar et al. (Am. J. Cardiol. 78 (1996), 284-287) sowie in EP-A-0 648 228 und WO 00/45176 beschrieben. Ein besonders bevorzugter Test ist ein Elektrochemilumineszenz-Immunoassay, z.B. das Testformat ECLIA^{®} von Roche Diagnostics GmbH, Mannheim.

Der Nachweis von IMA als nicht-kardiospezifischer Marker ist beispielsweise bei Wu et al. (Journal: MLO Medical Laboratory Observer 35 (2003), 36-38; 40) beschrieben. Der Nachweis von Myoglobin ist beispielsweise bei Pentillä et al. (Clin. Biochem. 33 (2002), 647-653) beschrieben. Der Nachweis von CD40 ist beispielsweise bei Heeschen et al. (New Engl. J. Med. 348 (2003), 1104-1111) beschrieben.

Bezüglich der Bestimmung von Troponin T als Beispiel für einen kardiospezifischen ischämischen Marker wird auf Katus et al. (Mol. Cell. Cardiol. 21 (1989), 1349-1353), Hamm et al. (N. Engl. Med. 327 (1992), 146-150), Ohmann et al. (N. Engl. J. Med. 335 (1996), 1333-1334), Christenson et al. (Clin. Chem. 44 (1998), 494-501) und zahlreiche andere Publikationen sowie auf EP-A-0 394 819 verwiesen. Besonders bevorzugte Tests zum Nachweis von Troponin T sind Elektrochemilumineszenz-Immunoassays, z.B. die Testformate Elecsys^{®} Troponin T- und Elecsys^{®} Troponin T STAT von Roche Diagnostics GmbH, Mannheim.

Eine besonders bevorzugte Kombination von Markern zur Diagnose von Myokardinfarkt ist ein neurohormonaler Marker, insbesondere NT-ProBNP, ein nicht-kardiospezifischer ischämischer Marker, insbesondere IMA, und mindestens ein nicht-kardiospezifischer myokalspezifischer Marker, insbesondere Myoglobin oder/und CK-MB. Ein signifikantes Risiko für Myokardinfarkt besteht, wenn mindestens drei der zuvor genannten Marker positiv sind. Dies gilt insbesondere auch für Patienten, die einen negativen Wert für einen kardiospezifischen ischämisch-nekrotischen Marker, wie Troponin T, aufweisen.

Eine weitere besonders bevorzugte Kombination von Markern zur Diagnose von Ischämie/instabiler Angina pectoris ist ein kardiospezifischer neurohormonaler Marker, insbesondere NT-ProBNP und ein nicht-kardiospezifischer ischämischer Marker, insbesondere IMA.

Ein weiterer Aspekt der Erfindung ist ein Reagenzienkit zur Diagnose von akuten myokardialen Erkrankungen, insbesondere von Myokardinfarkt, wobei der Reagenzienkit Nachweisreagenzien zur Bestimmung von mindestens zwei Markern enthält, wobei mindestens ein Nachweisreagenz für einen kardiospezifischen neurohormonalen Marker und ein Nachweisreagenz für ischämisch modifiziertes Albumin (IMA) als ein nicht-kardiospezifischer ischämischer Marker und gegebenenfalls ein Nachweisreagenz für einen nicht-kardiospezifischen muskelspezifischen Marker vorliegt. Gegebenenfalls kann weiterhin mindestens ein Nachweisreagenz für einen nicht-kardiospezifischen Marker für die Plättchenaktivierung oder/und einen kardiospezifischen ischämisch-nekrotischen Marker vorliegen.

Der Reagenzienkit ist vorzugsweise so ausgestaltet, dass er sich zur Durchführung von parallelen Bestimmungen der Marker und insbesondere zur Durchführung der Bestimmungen an einer einzigen Patientenprobe eignet. Hierfür ist es zweckmäßig, Nachweisreagenzien zu verwenden, die eine Bestimmung aller Marker durch ein einziges Testformat ermöglichen, beispielsweise einem Enzymuntest, einen Elektrochemilumineszenztest, einen turbidimetrischen Test oder einen Schnelltest auf einem Teststreifen,

Der Reagenzienkit kann so ausgestaltet sein,dass er sich zur Durchführung der Bestimmungen an einem Analyseautomaten oder Schnelltest eignet. Besonders bevorzugt liegen die Nachweisreagenzien für mehrere Marker auf einem einzigen Teststreifen vor.

In einer besonders bevorzugten Ausführungsform der Erfindung wird ein Reagenzienkit in Form eines Teststreifens zur Diagnose von akuten myokardialen Erkrankungen bereitgestellt, der Reagenzien zur Bestimmung von BNP oder NP-ProBNP, zur Bestimmung von IMA, gegebenenfalls zur Bestimmung von Myoglobin oder/und CK-MB und gegebenenfalls zur Bestimmung von Troponin T oder Troponin I, insbesondere zur Bestimmung von Troponin T, oder/und zur Bestimmung von CD40 enthält.

Das erfindungsgemäße Verfahren und der erfindungsgemäße Reagenzienkit können zur Identifizierung von Patienten mit akutem Koronarsyndrom eingesetzt werden, insbesondere zur Verbesserung der Früherkennung von akuten koronaren Ereignissen, beispielsweise zur Verbesserung der Früherkennung bei akutem Myokardinfarkt.

Weiterhin soll die vorliegende Erfindung durch das nachfolgende Beispiel erläutert werden.

### Beispiel

### Diagnosestellung von akuten myokardialen, ischämischen Erkrankungen durch Kombination von Markern

Die nachfolgend beschriebene Untersuchung belegt die Annahme, dass die Bestimmung mehrerer Marker - im Sinne der Erfindung - die Diagnosestellung von akuten myokardialen, ischämischen Erkrankungen verbessert. Am Beispiel von Brustschmerz-Patienten kann gezeigt werden, dass durch die Bestimmung mehrerer Marker, im Sinne der Erfindung, eine frühzeitigere Diagnose eines Myokardinfarktes (MI) bzw. einer ischämischen Erkrankung möglich ist.

### Studien Design:

40 Serum-Proben von Patienten, die mit Brustschmerzen in der Universitätsklinik in Lübeck aufgenommen wurden, wurden retrospektiv untersucht. Die Einschlusskriterien für Gruppe 1 (= MI Gruppe) waren ein negativer Troponin T Wert bei der Aufnahme und ein positiver Wert 4-6 Stunden danach. Gruppe 2 (= Kontrollgruppe) bestand aus Patienten mit negativen Troponin T Werten bei der Aufnahme und 4-6 Stunden danach. Die Proben wurden zufällig ausgewählt.

Die folgenden Marker wurden bestimmt:
- NT-proBNP: als kardiospezifischer neurohormonaler Marker
- IMA: als nicht-kardiospezifischer ischämischer Marker
- Myoglobin: als nicht-kardiospezifischer muskelspezifischer Marker
- CK-MB: als nicht-kardiospezifischer muskel spezifischer Marker

### Methode:

Die Proben wurden bei -20 °C gelagert und transportiert. NT-proBNP (Kat. Nr. 03121640) und Myoglobin (Kat. Nr. 1972146) wurden mit den entsprechenden Roche Reagenzien Kit auf dem Roche ELECSYS Analysenautomat gemäss den Herstellerangaben bestimmt. CK-MB und IMA wurden auf Hitachi 912 mit den Roche CK-MB Kit (Kat. Nr. 213284) und dem Ischemia Technologies ACB Kit (Kat. Nr. 01VAC260), entsprechend den Herstellerangaben bestimmt.

### Ergebnis:

Die Bestimmungsergebnisse aller Marker sind in Tabelle 1 zusammengefasst.

### a) Bestimmung eines kardiospezifischen neurohormonalen Markers (NT-proBNP) zusammen mit einem nicht-kardiospezifischen muskelspezifischen Marker (CK-MB)zur frühzeitigen Identifizierung von Patienten mit MI

Die folgenden Cut-off-Werte wurden zur Identifizierung eines positiven Wertes verwendet:

| | |
|---|---|
| NT-proBNP: | 350 pg/mL |
| CK-MB: | 12 U/L |

Betrachtet man die positiven Werte der beiden Marker einzeln und in Kombination, findet sich folgende Verteilung:

| Marker | NT-proBNP positive Werte | CK-MB positive Werte | NT-proBNP und CK-MB positive Werte |
|---|---|---|---|
| MI Gruppe (n=19) | 65 % | 35 % | 20 % |
| Kontrollgruppe (n=20) | 65 % | 15 % | 0 % |

NT-proBNP oder CK-MB alleine sind nicht spezifisch, um einen späteren Anstieg von Troponin T und damit einen Myokardinfarkt vorzeitig zu diagnostizieren. Betrachtet man jedoch die positiven Werte beider Marker, so ließe sich für 20 % der Patienten vorzeitig, d.h. bereits bei der Aufnahme, ein Myokardinfarkt diagnostizieren.

### b) Bestimmung aller vier Parameter zur vorzeitigen Identifizierung von Patienten mit MI

Hierzu wurden die folgenden cut-offs zur Identifizierung eines positiven Wertes verwendet:

| | |
|---|---|
| NT-proBNP: | 125 pg/mL |
| IMA: | 85 U/mL |
| Myoglobin: | 70 ng/mL |
| CK-MB: | 12 U/L |

18 der 20 Patienten der MI Gruppe haben bereits bei Aufnahme mindestens einen der vier Parameter positiv. In 65 % der Proben waren 0-2 Marker erhöht. In 35 % der getesteten Proben waren 3 oder 4 Marker über dem Cut-off-Wert.

In der Kontrollgruppe hat jedoch kein Patient 3 oder 4 Marker positiv. 10 % der Patienten hatten überhaupt keinen Anstieg eines der Marker, 30 % zeigten den Anstieg eines Markers und 60 % den Anstieg von zwei Markern zum Zeitpunkt der Aufnahme.

Das Ergebnis ist nochmals in der folgenden Tabelle zusammengefasst:

| Marker positiv | 0 | 1 | 2 | 3 | 4 |
|---|---|---|---|---|---|
| MI Gruppe (n=19) | 10 % | 25% | 30% | 20% | 15% |
| Kontrollgruppe (n=20) | 10% | 30% | 60% | 0% | 0% |

Durch Bestimmung aller vier Parameter und dem Kriterium, dass mindestens 3 der Marker erhöht sein müssen, ließe sich für 35 % der Patienten bereits bei der Aufnahme (alle Troponin negativ), ein Myokardinfarkt diagnostizieren.

### c) Bestimmung eines kardiospezifischen neurohormonalen Markers (NT-proBNP) zusammen mit einem nicht-kardiospezifischen ischämischen Marker (IMA) zur spezifischen Diagnose einer lschämie/instabilen Angina Pectoris

Ein positiver IMA Wert ist indikativ für eine Ischämie unklarer Genese. Betrachtet man das oben beschriebene Patientenkollektiv (Tabelle 1) hinsichtlich Ischämie, so wurde für 34 Patienten eine Diagnose gestellt (MI oder IAP), die zumeist eine ischämische Pathophysiologie aufweist. 6 Patienten hatten keine Ischämie als Enddiagnose (Patienten 23, 30, 32, 33, 36, 39 = Kontrollgruppe). 4 dieser Patienten zeigten jedoch einen erhöhten IMA Wert (Cut-off-Wert: 85 U/mL) (Patienten 23, 30, 32, 39 = "Falsch-Positive"). Bezieht man in die Diagnose noch einen positiven NT-proBNP Wert mit ein (Cut-off-Wert: 350 pg/mL) erhöht sich die Spezifität deutlich, d.h. es werden keine falsch-positiven Fälle mehr beobachtet. Dieses Ergebnis ist in der folgenden Tabelle zusammengefasst.

| Marker positiv | IMA | IMA + NT-proBNP |
|---|---|---|
| MI, IAP Gruppe (n=34) | 62 % | 29 % |
| Kontrollgruppe (n=6) | 67 % | 0 % |

**Tabelle 1: Werte der Marker des untersuchten Patientenkollektives**

| Diagnose | Pat.Nr/Probe | NT-proBNP [pg/mL] | IMA [U/mL] | Myoglobin ng/mL | CK-MB [U/L] |
|---|---|---|---|---|---|
| | | | | | |

| MI Gruppe (Bestätigter Herzinfarkt (1. Bestimmung Trop T negativ, 2. Bestimmung Trop T positiv) | | | | | |
|---|---|---|---|---|---|
| | | | | | |
| MI = Myokardinfarkt | 1 | 106 | 100 | 43 | 5 |
| MI | 2 | 398 | 80 | 29 | 5 |
| MI | 3 | 4663 | 107 | 207 | 23 |
| MI | 4 | 68 | 82 | 71 | -6 |
| MI | 5 | 11222 | 78 | 60 | 18 |
| MI | 6 | 105 | 120 | 43 | 5 |
| MI | 7 | 158 | 74 | 28 | 6 |
| MI | 8 | 16 | 115 | 32 | 6 |
| MI | 9 | 291 | 76 | 239 | 20 |
| MI | 10 | 15364 | 111 | 152 | 30 |
| MI | 11 | 147 | 100 | 107 | 3 |
| MI | 12 | 29 | 71 | 28 | 9 |
| MI | 13 | 189 | 110 | 955 | 32 |
| MI | 14 | 825 | 95 | 44 | 17 |
| MI | 15 | 62 | 101 | 98 | 6 |
| MI | 16 | 460 | 105 | 68 | 10 |
| MI | 17 | 1123 | 95 | 27 | 3 |
| MI | 18 | 154 | 94 | 48 | 6 |
| MI | 19 | 342 | 70 | 739 | 18 |
| MI | 20 | 79 | 80 | 35 | 3 |
| | | | | | |

| Kontrollgruppe (Kein Herzinfarkt (1.+2. Bestimmung Trop T negativ)) | | | | | |
|---|---|---|---|---|---|
| | | | | | |
| IAP = Instabile Angina Pectoris | 21 | 1125 | 83 | 17 | 10 |
| IAP | 22 | 4022 | 93 | 20 | 6 |
| Thoraxschmerz in Ruhe; BWS Syndrom | 23 | 212 | 100 | 25 | 8 |
| IAP | 24 | 35 | 84 | 23 | 7 |
| IAP | 25 | 458 | 97 | 33 | 8 |
| IAP | 26 | 84 | 92 | 50 | 5 |
| IAP | 27 | 2212 | 108 | 61 | 7 |
| IAP | 28 | 55 | 91 | 39 | 5 |
| IAP | 29 | 845 | 103 | 54 | 7 |
| Thoraxschmerz in Ruhe | 30 | 181 | 107 | 19 | 1 |
| IAP | 31 | 660 | 102 | 24 | 9 |
| Thoraxschmerz in Ruhe | 32 | 32 | 107 | 15 | 8 |
| Thoraxschmerz in Ruhe | 33 | 260 | 77 | 31 | 21 |
| IAP | 34 | 108 | 76 | 25 | 18 |
| IAP | 35 | 113 | 100 | 21 | 3 |
| li-th. Schmerz, rez. Gastr. | 36 | 98 | 65 | 20 | 7 |
| IAP | 37 | 328 | 66 | 35 | 13 |
| IAP | 38 | 192 | 103 | 48 | 6 |
| Thoraxschmerz in Ruhe; BWS Syndrom | 39 | 134 | 98 | 13 | 3 |
| IAP | 40 | 357 | 97 | 13 | 6 |

## Patentansprüche

1. Verfahren zur Diagnose von akuten myokardialen, ischämischen Erkrankungen,
**dadurch gekennzeichnet**
**dass** eine Bestimmung von mindestens zwei Markern an einer oder mehreren Proben aus einem zu untersuchenden Patienten durchgeführt wird, wobei jeweils mindestens ein kardiospezifischer neurohormonaler Marker und ischämisch modifiziertes Albumin (IMA) als nicht kardiospezifischer ischämischer Marker und gegebenenfalls ein nicht-kardiospezifischer muskelspezifischer Marker bestimmt wird.

2. Verfahren nach Anspruch 1,
**dadurch gekennzeichnet**
**dass** der kardiospezifische neurohormonale Marker ausgewählt wird aus A-Typ natriuretischem Peptid (ANP), dem N-terminalen Fragment von Pro-ANP (NT-ProANP), B-Typ natriuretischem Peptid (BNP) und dem N-terminalen Fragment von Pro-BNP (NT-ProBNP).

3. Verfahren nach Anspruch 2,
**dadurch gekennzeichnet**
**dass** BNP oder NT-ProBNP als neurohormonaler Marker bestimmt wird.

4. Verfahren nach einem der Ansprüche 1 bis 3,
**dadurch gekennzeichnet**
**dass** der nicht-kardiospezifische muskelspezifische Marker aus Myoglobin oder/und CK-MB ausgewählt wird.

5. Verfahren nach einem der Ansprüche 1 bis 4,
**dadurch gekennzeichnet**
**dass** zusätzlich ein nicht-kardiospezifischer Marker für die Plättchenaktivierung bestimmt wird.

6. Verfahren nach Anspruch 5,
**dadurch gekennzeichnet,**
**dass** CD40 als nicht-kardiospezifischer Marker für die Plättchenaktivierung bestimmt wird.

7. Verfahren nach einem der Ansprüche 1 bis 6,
**dadurch gekennzeichnet**
**dass** zusätzlich ein kardiospezifischer ischämisch-nekrotischer Marker bestimmt wird.

8. Verfahren nach Anspruch 7,
**dadurch gekennzeichnet**
**dass** der kardiospezifische ischämisch-nekrotische Marker aus Troponin T und Troponin 1 ausgewählt wird.

9. Verfahren nach Anspruch 8,
**dadurch gekennzeichnet**
**dass** Troponin T als ischämisch-nekrotischer Marker bestimmt wird.

10. Verfahren nach einem der Ansprüche 1 bis 9 zur Diagnose von akutem Myokardinfarkt.

11. Verfahren nach einem der Ansprüche 1 bis 10 zur Verbesserung der Früherkennung bei der Diagnose von akutem Myokardinfarkt.

12. Verfahren nach einem der Ansprüche 1 bis 11,
**dadurch gekennzeichnet**
**dass** parallele Bestimmungen der Marker durchgeführt werden.

13. Verfahren nach einem der Ansprüche 1 bis 12,
**dadurch gekennzeichnet**
**dass** die Bestimmungen an einer einzigen Patientenprobe durchgeführt werden.

14. Verfahren nach einem der Ansprüche 1 bis 13,
**dadurch gekennzeichnet**
**dass** die Bestimmungen an einem Analyseautomaten durchgeführt werden.

15. Verfahren nach einem der Ansprüche 1 bis 14,
**dadurch gekennzeichnet**
**dass** die Bestimmungen als Schnelltest durchgeführt werden.

16. Verfahren nach Anspruch 15,
**dadurch gekennzeichnet**
**dass** die Bestimmungen auf einem Teststreifen, insbesondere auf einem einzigen Teststreifen, durchgeführt werden.

17. Reagenzienkit zur Diagnose von akuten myokardialen, ischämischen Erkrankungen,
**dadurch gekennzeichnet**
**dass** er Nachweisreagenzien zur Bestimmung von mindestens zwei Markern enthält, wobei jeweils mindestens ein Nachweisreagenz für einen kardiospezifischen neurohormonalen Marker und ein Nachweisreagenz für ischämisch modifiziertes Albumin (IMA) als nicht kardiospezifischen ischämischen Marker und gegebenenfalls ein Nachweisreagenz für einen nicht-kardiospezifischen muskelspezifischen Marker vorliegt.

18. Reagenzienkit nach Anspruch 17,
**dadurch gekennzeichnet**
**dass** weiterhin mindestens ein Nachweisreagenz für einen nicht-kardiospezifischen Marker der Plättchenaktivierung oder/und einen kardiospezifischen ischämisch-nekrotischen Marker vorliegt.

19. Reagenzienkit nach Anspruch 17 oder 18 zur Durchführung von parallelen Bestimmungen der Marker.

20. Reagenzienkit nach einem der Ansprüche 17 bis 19 zur Durchführung der Bestimmungen in einer einzigen Patientenprobe.

21. Reagenzienkit nach einem der Ansprüche 17 bis 20 zur Durchführung der Bestimmungen an einem Analyseautomaten.

22. Reagenzienkit nach einem der Ansprüche 17 bis 20 zur Durchführung der Bestimmungen als Schnelltest.

23. Reagenzienkit nach Anspruch 22 zur Durchführung der Bestimmungen auf Teststreifen, insbesondere auf einem einzigen Teststreifen.

24. Reagenzienkit in Form eines Teststreifens zur Diagnose von akuten myokardialen, ischämischen Erkrankungen,
**dadurch gekennzeichnet,**
**dass** er Reagenzien zur Bestimmung von BNP oder NT-ProBNP, zur Bestimmung von IMA, gegebenenfalls zur Bestimmung von Myoglobin oder/und CK-MB und gegebenenfalls zur Bestimmung von Troponin T oder/und CD40 enthält.

25. Verwendung eines Reagenzienkits nach einem der Ansprüche 17 bis 24 zur Identifizierung von Patienten mit akutem Myokardinfarkt *in vitro.*

## Claims

1. Method for diagnosing acute myocardial, ischemic diseases
**characterized in that**
at least two markers are determined in one or more samples from a patient to be examined, where in each case at least one cardiospecific neurohormonal marker and ischemia-modified albumin (IMA) as a non-cardiospecific ischemic marker and optionally a non-cardiospecific muscle-specific marker are determined.

2. Method according to claim 1,
**characterized in that**
the cardiospecific neurohormonal marker is selected from A-type natriuretic peptide (ANP), the N-terminal fragment of pro-ANP (NT-proANP), B-type natriuretic peptide (BNP) and the N-terminal fragment of pro-BNP (NT-pro-BNP).

3. Method according to claim 2,
**characterized in that**
BNP or NT-proBNP is determined as the neurohormonal marker.

4. Method according to one of the claims 1 to 3,
**characterized in that**
the non-cardiospecific muscle-specific marker is selected from myoglobin or/and CK-MB.

5. Method according to one of the claims 1 to 4,
**characterized in that**
a non-cardiospecific marker for platelet activation is additionally determined.

6. Method according to claim 5,
**characterized in that**
CD40 is determined as a non-cardiospecific marker for platelet activation.

7. Method according to one of the claims 1 to 6,
**characterized in that**
a cardiospecific ischemic-necrotic marker is additionally determined.

8. Method according to claim 7,
**characterized in that**
the cardiospecific ischemic-necrotic marker is selected from troponin T and troponin I.

9. Method according to claim 8,
**characterized in that**
troponin T is determined as the ischemic-necrotic marker.

10. Method according to one of the claims 1 to 9 for the diagnosis of acute myocardial infarction.

11. Method according to one of the claims 1 to 10 for improving the early detection when diagnosing acute myocardial infarction.

12. Method according to one of the claims 1 to 11,
**characterized in that**
the markers are determined in parallel.

13. Method according to one of the claims 1 to 12,
**characterized in that**
the determinations are carried out on a single patient sample.

14. Method according to one of the claims 1 to 13,
**characterized in that**
the determinations are carried out on an automated analyzer.

15. Method according to one of the claims 1 to 14,
**characterized in that**
the determinations are carried out as a rapid test.

16. Method according to claim 15,
**characterized in that**
the determinations are carried out on a test strip and in particular on a single test strip.

17. Reagent kit for the diagnosis of acute myocardial, ischemic diseases,
**characterized in that**
it contains detection reagents for determining at least two markers, where in each case at least one detection reagent for a cardiospecific neurohormonal marker and a detection reagent for ischemia-modified albumin (IMA) as a non-cardiospecific ischemic marker and optionally a detection reagent for a non-cardiospecific, muscle-specific marker are present.

18. Reagent kit according to claim 17,
**characterized in that**
at least one detection reagent for a non-cardiospecific marker of platelet activation or/and for a cardiospecific ischemic-necrotic marker are additionally present.

19. Reagent kit according to claim 17 or 18 for carrying out concurrent determinations of the markers.

20. Reagent kit according to one of the claims 17 to 19 for carrying out the determinations on a single patient sample.

21. Reagent kit according to one of the claims 17 to 20 for carrying out the determinations on an automated analyzer.

22. Reagent kit according to one of the claim 17 to 20 for carrying out the determinations as a rapid test.

23. Reagent kit according to claim 22 for carrying out the determinations on test strips, in particular on a single test strip.

24. Reagent kit in the form of a test strip for diagnosing acute myocardial, ischemic diseases,
**characterized in that**
it contains reagents for determining BNP or NP-proBNP, for determining IMA, optionally for determining myoglobin or/and CK-MB and optionally for determining troponin T or/and CD40.

25. Use of a reagent kit according to one of the claims 17 to 24 for identifying patients with acute myocardial infarction *in vitro.*

## Revendications

1. Procédé de diagnostic de maladies aiguës ischémiques du myocarde,
**caractérisé en ce qu'**on
effectue une détermination d'au moins deux marqueurs sur un ou plusieurs échantillons provenant d'un patient à examiner, dans lequel on détermine respectivement au moins un marqueur neurohormonal cardiospécifique et l'albumine modifiée par ischémie (IMA) comme marqueur ischémique non cardiospécifique et éventuellement un marqueur non cardiospécifique spécifique des muscles.

2. Procédé selon la revendication 1,
**caractérisé en ce que**
le marqueur neurohormonal cardiospécifique est choisi parmi peptide natriurétique de type A (ANP), le fragment N-terminal de Pro-ANP (NT-ProANP), peptide natriurétique de type B (BNP) et le fragment N-terminal de Pro-BNP (NT-ProBNP).

3. Procédé selon la revendication 2,
**caractérisé en ce que**
BNP ou NT-ProBNP est déterminé comme marqueur neurohormonal.

4. Procédé selon l'une des revendications 1 à 3,
**caractérisé en ce que**
le marqueur non cardiospécifique spécifique des muscles est choisi parmi myoglobine et/ou CK-MB.

5. Procédé selon l'une des revendications 1 à 4,
**caractérisé en ce qu'**on
détermine additionnellement un marqueur non cardiospécifique pour l'activation des plaquettes.

6. Procédé selon la revendication 5,
**caractérisé en ce qu'**on
détermine CD40 comme marqueur non cardiospécifique pour l'activation des plaquettes.

7. Procédé selon l'une des revendications 1 à 6,
**caractérisé en ce qu'**on
détermine additionnellement un marqueur cardiospécifique nécrotique par ischémie.

8. Procédé selon la revendication 7,
**caractérisé en ce qu'**on
choisit le marqueur cardiospécifique nécrotique par ischémie parmi troponine T et troponine I.

9. Procédé selon la revendication 8,
**caractérisé en ce qu'**on
détermine la troponine T comme marqueur nécrotique par ischémie.

10. Procédé selon l'une des revendications 1 à 9 pour le diagnostic de l'infarctus aigu du myocarde.

11. Procédé selon l'une des revendications 1 à 10 pour l'amélioration de la reconnaissance précoce dans le diagnostic de l'infarctus aigu du myocarde.

12. Procédé selon l'une des revendications 1 à 11,
**caractérisé en ce qu'**on
effectue des déterminations parallèles des marqueurs.

13. Procédé selon l'une des revendications 1 à 12,
**caractérisé en ce qu'**on
effectue les déterminations sur un échantillon unique du patient.

14. Procédé selon l'une des revendications 1 à 13,
**caractérisé en ce qu'**on
effectue les déterminations sur un analyseur automatique.

15. Procédé selon l'une des revendications 1 à 14,
**caractérisé en ce qu'**on
effectue les déterminations sous forme de test rapide.

16. Procédé selon la revendication 15,
**caractérisé en ce qu'**on
effectue les déterminations sur un ruban de test, en particulier sur un seul ruban de test.

17. Ensemble de réactifs destiné au diagnostic de maladies aiguës ischémiques du myocarde,
**caractérisé en ce qu'**il
contient des réactifs de détection pour la détermination d'au moins deux marqueurs, dans lequel sont présents respectivement un réactif de détection pour un marqueur neurohormonal cardiospécifique et un réactif de détection pour l'albumine modifiée par ischémie (IMA) comme marqueur ischémique non cardiospécifique et éventuellement un réactif de détection pour un marqueur non cardiospécifique spécifique des muscles.

18. Ensemble de réactifs selon la revendication 17,
**caractérisé en ce que**
sont présents en outre au moins un réactif de détection pour un marqueur non cardiospécifique de l'activation des plaquettes et/ou un marqueur cardiospécifique nécrotique par ischémie.

19. Ensemble de réactifs selon la revendication 17 ou 18 destiné à la réalisation de déterminations parallèles des marqueurs.

20. Ensemble de réactifs selon l'une des revendications 17 à 19 destiné à la réalisation des déterminations dans un échantillon unique du patient.

21. Ensemble de réactifs selon l'une des revendications 17 à 20 destiné à la réalisation des déterminations sur un analyseur automatique.

22. Ensemble de réactifs selon l'une des revendications 17 à 20 pour la réalisation des déterminations sous la forme de test rapide.

23. Ensemble de réactifs selon la revendication 22 destiné à la réalisation des déterminations sur des rubans de test, en particulier sur un ruban de test unique.

24. Ensemble de réactifs sous la forme d'un ruban de test destiné au diagnostic de maladies aiguës ischémiques du myocardé,
**caractérisé en ce qu'**il
contient des réactifs destinés à la détermination de BNP ou NT-ProBNP, destinés à la détermination de IMA, éventuellement destinés à la détermination de la myoglobine et/ou de CK-MB et éventuellement destinés à la détermination de la troponine T et/ou de CD40.

25. Utilisation d'un ensemble de réactifs selon l'une des revendications 17 à 24 pour l'identification *in vitro* de patients avec un infarctus aigu du myocarde.
